(19) Europäisches Patentamt
European Patent Office
Office européen des brevets

(11) Veröffentlichungsnummer: 0 327 899
A2

(12) # EUROPÄISCHE PATENTANMELDUNG

(21) Anmeldenummer: 89101390.6

(22) Anmeldetag: 27.01.89

(51) Int. Cl.⁴: C07D 249/08 , C07D 233/60 , A01N 43/50 , A01N 43/653

(30) Priorität: 09.02.88 DE 3803832

(43) Veröffentlichungstag der Anmeldung: 16.08.89 Patentblatt 89/33

(84) Benannte Vertragsstaaten: BE CH DE FR GB IT LI NL

(71) Anmelder: BAYER AG
D-5090 Leverkusen 1 Bayerwerk(DE)

(72) Erfinder: Elbe, Hans-Ludwig, Dr.
Dasnöckel 59
D-5600 Wuppertal 11(DE)
Erfinder: Büchel, Karl Heinz, Prof. Dr.
Dabringhausener Strasse 42
D-5093 Burscheid(DE)
Erfinder: Dutzmann, Stefan, Dr.
Leinenweberweg 33
D-4000 Düsseldorf 13(DE)
Erfinder: Reinecke, Paul, Dr.
Steinstrasse 8
D-5090 Leverkusen 3(DE)

(54) **Substituierte Vinylazole.**

(57) Neue substituierte Vinylazole der Formel

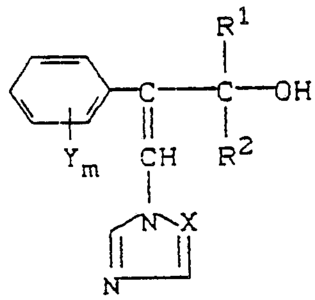

(I)

in welcher
$R^1$ für Alkyl steht,
$R^2$ für Alkyl steht,
X für ein Stickstoffatom oder die CH-Gruppe steht,
Y für Halogen, Alkyl, Cycloalkyl, Alkoxy, Alkylthio, Halogenalkyl, Halogenalkoxy, Halogenalkylthio, gegebenenfalls substituiertes Phenyl, gegebenenfalls substituiertes Phenoxy, gegebenenfalls substituiertes Phenylalkyl und/oder gegebenenfalls substituiertes Phenylalkoxy steht und
m für die Zahlen 0, 1, 2 oder 3 steht,
sowie deren Säureadditions-Salze und Metallsalz-Komplexe, ein Verfahren zur Herstellung der neuen Stoffe und deren Verwendung als Fungizide.

EP 0 327 899 A2

## Substituierte Vinylazole

Die vorliegende Erfindung betrifft neue substituierte Vinylazole, ein Verfahren zu ihrer Herstellung und ihre Verwendung als Fungizide.

Es ist bereits bekannt geworden, daß bestimmte Hydroxyalkylazole fungizide Eigenschaften aufweisen (vergleiche DE-OS 32 45 504). So können z.B. 2,4-Bis-(4-chlorphenyl)-3,3-dimethyl-1-(1,2,4-triazol-1-yl)-2-butanol und 3-(4-Chlor-2-methylphenoxy)-2-(4-chlorphenyl)-3,3-dimethyl-1-(1,2,4-triazol-1-yl)-2-propanol zur Bekämpfung von Pilzen eingesetzt werden. Die Wirksamkeit dieser Stoffe ist jedoch, vor allem bei niedrigen Aufwandmengen und -konzentrationen, nicht immer ganz befriedigend.

Es wurden nun neue substituierte Vinylazole der Formel

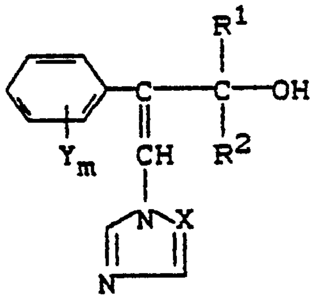

(I)

in welcher

$R^1$ für Alkyl steht,

$R^2$ für Alkyl steht,

X für ein Stickstoffatom oder die CH-Gruppe steht,

Y für Halogen, Alkyl, Cycloalkyl, Alkoxy, Alkylthio, Halogenalkyl, Halogenalkoxy, Halogenalkylthio, gegebenenfalls substituiertes Phenyl, gegebenenfalls substituiertes Phenoxy, gegebenenfalls substituiertes Phenylalkyl und/oder gegebenenfalls substituiertes Phenylalkoxy steht und

m für die Zahlen 0, 1, 2 oder 3 steht,

sowie deren Säureadditions-Salze und Metallsalz-Komplexe gefunden.

Die neuen substituierten Vinylazole der Formel (I) besitzen für den Fall, daß $R^1$ und $R^2$ voneinander verschieden sind, ein asymmetrisch substituiertes Kohlenstoffatom; sie können deshalb in Form von Enantiomeren vorliegen. Die Verbindungen der Formel (I) können außerdem in zwei geometrischen Isomerenformen vorliegen, je nach Anordnung der Gruppen, die an die Doppelbindung gebunden sind. Die Erfindung betrifft sowohl die Isomerengemische als auch die einzelnen Isomeren.

Weiterhin wurde gefunden, daß man substituierte Vinylazole der Formel (I) bzw. deren Säureadditions-Salze und Metallsalz-Komplexe erhält, wenn man Oxirane der Formel

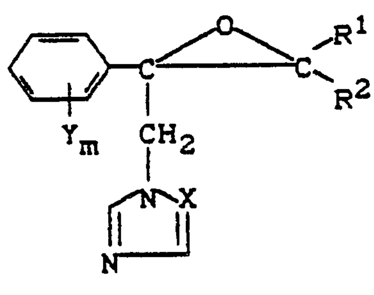

(II)

in welcher

$R^1$, $R^2$, X, Y und m die oben angegebene Bedeutung haben,

mit Wasser gegebenenfalls in Gegenwart eines Verdünnungsmittels und in Gegenwart starker Säuren umsetzt und gegebenenfalls anschließend an die so erhaltenen Verbindungen der Formel (I) eine Säure oder ein Metallsalz addiert.

Schließlich wurde gefunden, daß die neuen substituierten Vinylazole der Formel (I) sowie deren

Säureadditions-Salze und Metallsalz-Komplexe sehr gute fungizide Eigenschaften besitzen.

Überraschenderweise zeichnen sich die erfindungsgemäßen Stoffe durch eine bessere fungizide Wirkung aus, als konstitutionell ähnliche, aus dem Stand der Technik bekannte Verbindungen gleicher Indikation. So übertreffen die erfindungsgemäßen Stoffe zum Beispiel 2,4-Bis-(4-chlorphenyl)-3,3-dimethyl-1-(1,2,4-triazol-1-yl)-2-butanol und 3-(4-Chlor-2-methylphenoxy)-2-(4-chlorphenyl)-3,3-dimethyl-1-(1,2,4-triazol-1-yl)-2-propanol bezüglich ihrer fungiziden Eigenschaften.

Außerdem sind die neuen substituierten Vinylazole der Formel (I) interessante Zwischenprodukte zur Herstellung von weiteren Pflanzenschutz-Wirkstoffen. So können durch Umsetzungen an der Hydroxy-Gruppe zum Beispiel Ester und Ether erhalten werden. Weiterhin können durch Umsetzungen mit z.B. Acylhalogeniden oder Carbamoylchloriden Acyl- oder Carbamoyl-Derivate der Verbindungen der Formel (I) erhalten werden.

Die erfindungsgemäßen substituierten Vinylazole sind durch die Formel (I) allgemein definiert. Bevorzugt sind Verbindungen der Formel (I), in denen

$R^1$ für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht,

X für ein Stickstoffatom oder die CH-Gruppe steht,

Y für Halogen, Alkyl mit 1 bis 4 Kohlenstoffatomen, Cycloalkyl mit 3 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, insbesondere Fluor- und/oder Chloratomen, Halogenalkoxy mit 1 oder 2 Kohlenstoffatomen und 1 bis 5 gleichen oder verschiedenen Halogenatomen, insbesondere Fluor- und/oder Chloratomen, Halogenalkylthio mit 1 oder 2 Kohlenstoffatomen und 1 bis 5 gleichen oder verschiedenen Halogenatomen, insbesondere Fluor- und/oder Chloratomen, sowie für gegebenenfalls durch Halogen und/oder Alkyl mit 1 oder 2 Kohlenstoffatomen substituiertes Phenyl, gegebenenfalls durch Halogen und/oder Alkyl mit 1 oder 2 Kohlenstoffatomen substituiertes Phenoxy, gegebenenfalls durch Halogen und/oder Alkyl mit 1 oder 2 Kohlenstoffatomen substitu iertes Phenylalkyl mit 1 oder 2 Kohlenstoffatomen im Alkylteil und/oder für gegebenenfalls durch Halogen und/oder Alkyl mit 1 oder 2 Kohlenstoffatomen substituiertes Phenylalkoxy mit 1 oder 2 Kohlenstoffatomen im Alkoxyteil steht, und

m für die Zahlen 0, 1, 2 oder 3 steht.

Wenn m für die Zahlen 2 oder 3 steht, können die Bedeutungen von Y gleich oder verschieden sein.

Besonders bevorzugt sind diejenigen Verbindungen der Formel (I), in denen

$R^1$ für Methyl oder Ethyl steht;

$R^2$ für Methyl oder Ethyl steht;

X für ein Stickstoffatom oder die CH-Gruppe steht;

Y für Fluor, Chlor, Brom, Methyl, Isopropyl, tert.-Butyl, Cyclohexyl, Methoxy, Methylthio, Trifluormethyl, Trifluormethoxy, Trifluormethylthio sowie für gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl und/oder für gegebenenfalls durch Fluor, Chlor und/oder Methyl substituiertes Benzyloxy steht und

m für die Zahlen 0, 1, 2 oder 3 steht.

Bevorzugte erfindungsgemäße Verbindungen sind auch Additionsprodukte aus Säuren und denjenigen substituierten Vinylazolen der Formel (I), in denen die Substituenten $R^1$, $R^2$, X und Y under der Index m die Bedeutungen haben, die bereits vorzugsweise für diese Substituenten und diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure sowie Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen substituierten Vinylazolen der Formel (I), in denen die Substituenten $R^1$, $R^2$, X und Y und der Index m die Bedeutungen haben, die bereits vorzugsweise für diese Substituenten und diesen Index genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhand die Halogenwasserstoffsäuren, wie z.B. die Chlorwasserstoffsäure und die Bromwasserstoffsäure, Salpetersäure und Schwefelsäure.

Als Beispiele für substituierte Vinylazole der Formel (I) seien im einzelnen die in der folgenden Tabelle aufgeführten Stoffe genannt.

**Tabelle 1:**

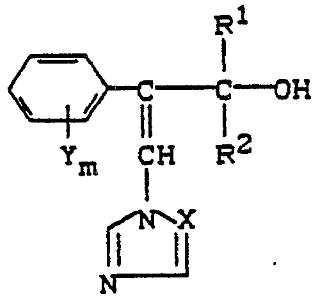 (I)

| $Y_m$ | $R^1$ | $R^2$ | X |
|---|---|---|---|
| 4-Br | $CH_3$ | $CH_3$ | N(CH) |
| 2-Br | $CH_3$ | $CH_3$ | " |
| 4-$OCH_3$ | $CH_3$ | $CH_3$ | " |
| 4 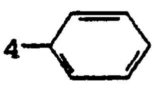 | $CH_3$ | $CH_3$ | " |
| 4- 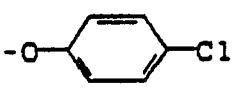 | $CH_3$ | $CH_3$ | " |
| 4-$CH_3$ | $CH_3$ | $CH_3$ | " |
| 4 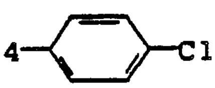 | $CH_3$ | $CH_3$ | " |

Tabelle 1: (Fortsetzung)

| $Y_m$ | $R^1$ | $R^2$ | X |
|---|---|---|---|
| 4 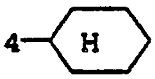 | $CH_3$ | $CH_3$ | N(CH) |
| 4-$SCH_3$ | $CH_3$ | $CH_3$ | " |
| 4-$OCHF_2$ | $CH_3$ | $CH_3$ | " |
| 4-$OCF_2Cl$ | $CH_3$ | $CH_3$ | " |
| 3-Cl, 4-$OCHF_2$ | $CH_3$ | $CH_3$ | " |
| 3-Cl, 4-$OCF_2Cl$ | $CH_3$ | $CH_3$ | " |
| 4-$SCHF_2$ | $CH_3$ | $CH_3$ | " |
| 2,4-$F_2$ | $CH_3$ | $CH_3$ | " |
| 2-F, 4-$CF_3$ | $CH_3$ | $CH_3$ | " |
| 2-F, 4-$OCF_3$ | $CH_3$ | $CH_3$ | " |
| 2-F, 4-$SCF_3$ | $CH_3$ | $CH_3$ | " |
| 4-$OCF_3$ | $CH_3$ | $CH_3$ | " |
| 4-$SCF_3$ | $CH_3$ | $CH_3$ | " |
| 4-F | $CH_3$ | $CH_3$ | " |
| 3-F | $CH_3$ | $CH_3$ | " |
| 2-F | $CH_3$ | $CH_3$ | " |
| 2-Cl, 4-F | $CH_3$ | $CH_3$ | " |
| 2-F, 4-Cl | $CH_3$ | $CH_3$ | " |
| 3-Cl, 4-$OCF_3$ | $CH_3$ | $CH_3$ | " |
| 3-Cl, 4-$SCF_3$ | $CH_3$ | $CH_3$ | " |

<u>Tabelle 1:</u> (Fortsetzung)

| $Y_m$ | $R^1$ | $R^2$ | X |
|---|---|---|---|
| 3,4-$Cl_2$ | $CH_3$ | $CH_3$ | N(CH) |
| 2,4-$Cl_2$ | $CH_3$ | $CH_3$ | " |
| 3,4,6-$Cl_3$ | $CH_3$ | $CH_3$ | " |
| 3,4,6-$F_3$ | $CH_3$ | $CH_3$ | " |
| 3-F, 4,6-$Cl_2$ | $CH_3$ | $CH_3$ | " |
| 4-Br | $CH_3$ | $C_2H_5$ | " |
| 2-Br | $CH_3$ | $C_2H_5$ | " |
| 4-$OCH_3$ | $CH_3$ | $C_2H_5$ | " |
| 4- 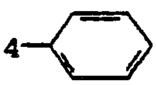 | $CH_3$ | $C_2H_5$ | " |
| 4- 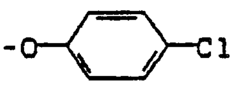 | $CH_3$ | $C_2H_5$ | " |
| 4-$CH_3$ | $CH_3$ | $C_2H_5$ | " |
| 4- 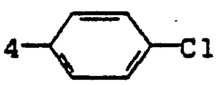 | $CH_3$ | $C_2H_5$ | " |
| 4- 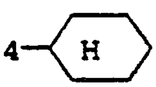 | $CH_3$ | $C_2H_5$ | " |
| 4-$SCH_3$ | $CH_3$ | $C_2H_5$ | " |
| 4-$OCHF_2$ | $CH_3$ | $C_2H_5$ | " |
| 4-$OCF_2Cl$ | $CH_3$ | $C_2H_5$ | " |
| 3-Cl, 4-$OCHF_2$ | $CH_3$ | $C_2H_5$ | " |
| 3-Cl, 4-$OCF_2Cl$ | $CH_3$ | $C_2H_5$ | " |
| 4-$SCHF_2$ | $CH_3$ | $C_2H_5$ | " |
| 4-Cl | $CH_3$ | $C_2H_5$ | " |

Tabelle 1: (Fortsetzung)

| $Y_m$ | $R^1$ | $R^2$ | X |
|---|---|---|---|
| 3-Cl | $CH_3$ | $C_2H_5$ | N(CH) |
| 2-Cl | $CH_3$ | $C_2H_5$ | " |
| 2,4-$F_2$ | $CH_3$ | $C_2H_5$ | " |
| 2-F, 4-$CF_3$ | $CH_3$ | $C_2H_5$ | " |
| 2-F, 4-$OCF_3$ | $CH_3$ | $C_2H_5$ | " |
| 2-F, 4-$SCF_3$ | $CH_3$ | $C_2H_5$ | " |
| 4-$OCF_3$ | $CH_3$ | $C_2H_5$ | " |
| 4-$SCF_3$ | $CH_3$ | $C_2H_5$ | " |
| 4-F | $CH_3$ | $C_2H_5$ | " |
| 3-F | $CH_3$ | $C_2H_5$ | " |
| 2-F | $CH_3$ | $C_2H_5$ | " |
| 2-Cl, 4-F | $CH_3$ | $C_2H_5$ | " |
| 2-F, 4-Cl | $CH_3$ | $C_2H_5$ | " |
| 3-Cl, 4-$OCF_3$ | $CH_3$ | $C_2H_5$ | " |
| 3-Cl, 4-$SCF_3$ | $CH_3$ | $C_2H_5$ | " |
| 3,4-$Cl_2$ | $CH_3$ | $C_2H_5$ | " |
| 2,4-$Cl_2$ | $CH_3$ | $C_2H_5$ | " |
| 3,4,6-$Cl_3$ | $CH_3$ | $C_2H_5$ | " |
| 3,4,6-$F_3$ | $CH_3$ | $C_2H_5$ | " |
| 3-F, 4,6-$Cl_2$ | $CH_3$ | $C_2H_5$ | " |

Tabelle 1: (Fortsetzung)

| $Y_m$ | $R^1$ | $R^2$ | X |
|---|---|---|---|
| 4-Cl | $C_2H_5$ | $C_2H_5$ | N(CH) |
| 3-Cl | $C_2H_5$ | $C_2H_5$ | " |
| 2-Cl | $C_2H_5$ | $C_2H_5$ | " |

Verwendet man beispielsweise 3,3-Dimethyl-2-(4-trifluormethoxy-phenyl)-2-(1,2,4-triazol-1-yl-methyl)-oxiran als Ausgangsstoff und Salzsäure als starke Säure, so kann der Ablauf des erfindungsgemäßen Verfahrens durch das folgende Formelschema wiedergegeben werden:

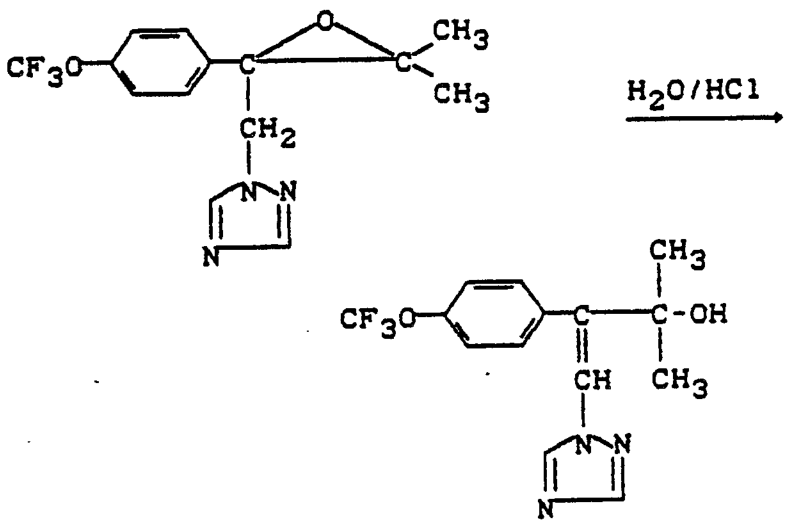


Die bei der Durchführung des erfindungsgemäßen Verfahrens als Ausgangsstoffe benötigten Oxirane sind durch die Formel (II) allgemein definiert. In dieser Formel haben $R^1$, $R^2$, X, Y und der Index m vorzugsweise diejenigen Bedeutungen, die bereits im Zusammenhang mit der Beschreibung der erfindungsgemäßen Stoffe der Formel (I) vorzugsweise für diese Reste und diesen Index genannt wurden.

Die Oxirane der Formel (II) sind noch nicht bekannt. Sie lassen sich herstellen, index man Hydroxyalkylazole der Formel

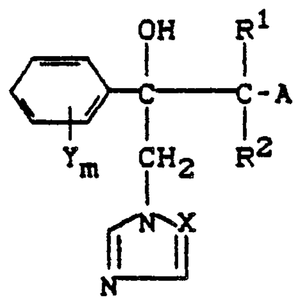

(III)

in welcher
$R^1$, $R^2$, X, Y und m die oben angegebene Bedeutung haben und
A für eine nucleophile Abgangsgruppe steht
gegebenenfalls in Gegenwart eines Verdünnungsmittels mit starken Basen umsetzt.

Die bei der Durchführung des obigen Verfahrens zur Herstellung der Oxirane der Formel (II) als Ausgangsstoffe benötigten Hydroxyalkylazole sind durch die Formel (III) allgemein definiert. In dieser Formel haben $R^1$, $R^2$, X, Y und der Index m vorzugsweise diejenigen Bedeutungen, die bereits im Zusammenhang mit der Beschreibung der erfindungsgemäßen Stoffe der Formel (I) vorzugsweise für diese Reste und diesen Index genannt wurden.

A steht vorzugsweise für einen Phenolat-, 4-Chlorphenolat- oder Thiophenolat-Rest.

Die Hydroxyalkylazole der Formel (III) sind bekannt oder können nach literaturbekannten Verfahren hergestellt werden (vergl. DE-OS 32 45 504).

Als Verdünnungsmittel können bei der Durchführung des obigen Verfahrens zur Herstellung der Oxirane der Formel (II) alle üblichen inerten organischen Solventien eingesetzt werden. Vorzugsweise verwendbar sind Ether, wie Tetrahydrofuran und Dioxan; aromatische Kohlenwasserstoffe, wie Toluol und Xylol und aliphatische und cycloaliphatische Kohlenwasserstoffe, wie Hexan und Cyclohexan.

Als Basen können bei der Durchführung des obigen Verfahrens zur Herstellung von Oxiranen der Formel (II) alle üblicherweise für derartige Umsetzungen verwendbaren starken Basen eingesetzt werden. Vorzugsweise in Frage kommen Alkalimetallhydroxide wie z. B. Natrium- und Kaliumhydroxid, Alkalimetallalkoholate wie Natrium- und Kaliummethylat, -ethylat, n-Propylat und t-Butylat, sowie Hydride wie z. B. Natriumhydrid.

Die Reaktionstemperaturen können bei der Durchführung des obigen Verfahrens zur Herstellung von Oxiranen der Formel (II) in einem größeren Bereich variiert werden. Im allgemeinen arbeitet man bei Temperaturen zwischen 0 °C und 150 °C, vorzugsweise zwischen 20 °C und 100 °C. Die Reaktionszeit beträgt im allgemeinen 2 bis 24 Stunden, vorzugsweise 2 bis 10 Stunden.

Bei der Durchführung des obigen Verfahrens zur Herstellung von Oxiranen der Formel (II) setzt man auf 1 Mol an Hydroxyalkylazol der Formel (III) im allgemeinen 1 bis 2 Mol, vorzugsweise 1 bis 1,2 Mol Base ein.

Die Isolierung der Verbindungen der Formel (II) erfolgt nach üblichen Methoden. Im allgemeinen geht man so vor, daß man das Reaktionsgemisch mit Wasser versetzt, dann mit einem mit Wasser wenig mischbaren organischen Lösungsmittel extrahiert und die vereinigten organischen Phasen nach gegebenenfalls vorherigem Waschen mit Wasser einengt. Das anfallende Produkt kann gegebenenfalls nach üblichen Methoden, zum Beispiel auf chromatographischem Wege, gereinigt werden.

Bei der Druchführung des erfindungsgemäßen Verfahrens kommen als Verdünnungsmittel alle üblichen inerten organischen Solventien in Frage. Vorzugsweise verwendbar sind Ether, wie Tetrahydrofuran und Dioxan; aromatische Kohlenwasserstoffe, wie Toluol und Xylol und aliphatische und cycloaliphatische Kohlenwasserstoffe, wie Hexan und Cyclohexan.

Das erfindungsgemäße Verfahren wird in Gegenwart starker anorganischer oder organischer Säuren durchgeführt. Vorzugsweise verwendbar sind Salzsäure, Salpetersäure, Schwefelsäure, Trifluoressigsäure, Toluolsulfonsäure und Naphthalinsulfonsäure.

Die Reaktionstemperaturen können bei der Durchführung des erfindungsgemäßen Verfahrens in einem größeren Bereich variiert werden. Im allgemeinen arbeitet man bei Temperaturen zwischen 20 °C und 150 °C, vorzugsweise bei Temperaturen zwischen 25 °C und 100 °C. Die Reaktionszeit beträgt im allgemeinen 2 bis 24 Stunden, vorzugsweise 2 bis 10 Stunden.

Das erfindungsgemäße Verfahren wird im allgemeinen unter Normaldruck durchgeführt, gegebenenfalls auch unter erhöhtem Druck.

Bei der Druchführung des erfindungsgemäßen Verfahrens setzt man auf 1 Mol Oxiran der Formel (II) im allgemeinen 0,1 bis 10 Mol, vorzugsweise 0,1 bis 5 Mol Säure ein. Es ist besonders vorteilhaft, die Verbindungen der Formel (II) herzustellen und ohne Zwischenisolierung weiter umzusetzen.

Die Isolierung der Verbindungen der Formel (I) erfolgt nach üblichen Methoden. Im allgemeinen geht man so vor, daß man den Säureüberschuß nach beendeter Umsetzung mit schwachen Basen neutralisiert, dann das Reaktionsgemisch mit einem mit Wasser nur wenig mischbaren organischen Lösungsmittel extrahiert und die vereinigten organischen Phasen nach gegebenenfalls vorherigem Waschen mit Wasser unter vermindertem Druck einengt. Das anfallende Produkt kann gegebenenfalls nach üblichen Methoden, zum Beispiel auf chromatographischem Wege, gereinigt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen

Lösungsmittel gereinigt werden.

Zur Herstellung von Metallsalz-Komplexen der Verbindungen der Formel (I) kommen vorzugsweise diejenigen Salze von Metallen in Frage, die bereits weiter oben im Zusammenhang mit der Beschreibung der erfindungsgemäßen Metallsalz-Komplexe als bevorzugte Metallsalze genannt wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol, und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide zur Bekämpfung von unerwünschten Mikroorganismen praktisch eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg bei der Saatgutbehandlung bei Gerste einsetzen. Außerdem besitzen die erfindungsgemäßen Wirkstoffe auch eine gute fungizide Wirkung gegen Getreiderost, Pyrenophora teres, Apfelschorf, echte Mehltaupilze im Obst-und Getreidebau, Pyricularia oryzae und Pellicularia sasakii an Reis.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellung und Verwendung der erfindungsgemäßen Wirkstoffe gehen aus den folgenden Beispielen hervor.

## Herstellungsbeispiele

### Beispiel 1

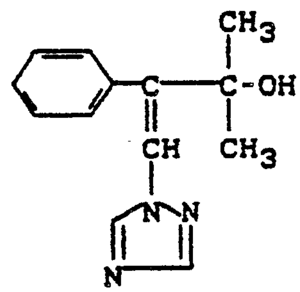

15 g (0,042 Mol) 3-(4-Chlorphenoxy)-2-phenyl-3-methyl-1-(1,2,4-triazol-1-yl)-2-butanol werden in 90 ml Dioxan vorgelegt und bei Raumtemperatur portionsweise mit 1,4 g (0,045 Mol) Natriumhydrid (80 %ig) versetzt. Danach wird das Reaktionsgemisch 10 Stunden bei Rückflußtemperatur gerührt. Man läßt das Reaktionsgemisch auf Raumtemperatur abkühlen, versetzt mit 50 ml Wasser und 2 ml konzentrierter Salzsäure und läßt 1 Stunde nachrühren. Anschließend wird das Reaktionsgemisch mit Natriumbicarbonat neutralisiert und mit Dichlormethan extrahiert. Die organische Phase wird über Natriumsulfat getrocknet und unter vermindertem Druck eingeengt. Der Rückstand wird an Kieselgel mit einem Gemisch aus Essigester/Cyclohexan = 3 : 1 als Laufmittel chromatographiert.

Man erhält 4,7 g (48,9 % der Theorie) an 3-Hydroxy-3-methyl-2-phenyl-1-(1,2,4-triazol-1-yl)-but-1-en vom Schmelzpunkt 122° C.

In analoger Weise zu Beispiel 1 und unter Berücksichtigung der Angaben zu dem erfindungsgemäßen Verfahren, werden die in der folgenden Tabelle 2 aufgeführten Verbindungen der Formel (I) hergestellt:

**Tabelle 2:**

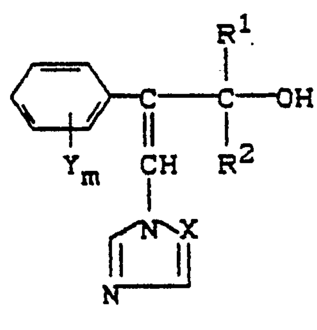

(I)

| Bsp. Nr. | $R^1$ | $R^2$ | X | 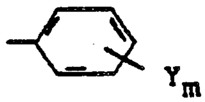 | physikal. Konstante |
|---|---|---|---|---|---|
| 2 | $CH_3$ | $CH_3$ | N | 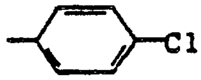 | Fp=94° C |
| 3 | $CH_3$ | $CH_3$ | N | 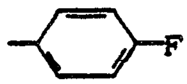 | $^1$H-NMR*): δ=1,45(s);6H δ=2,35(s);1H |
| 4 | $CH_3$ | $CH_3$ | N | 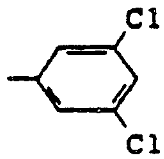 | Fp=149° C |
| 5 | $CH_3$ | $CH_3$ | N | 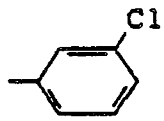 | Fp=127° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung von Oxiranen der Formel (II)

Beispiel 6

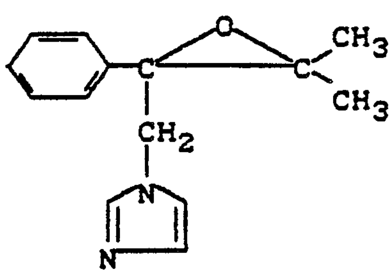

(II-1)

9 g (0,0253 Mol) 3,3-Dimethyl-3-(4-chlorphenoxy)-2-phenyl-1-(imidazol-1-yl)-2-propanol werden bei Raumtemperatur in 50 ml Dioxan gelöst. Man gibt 0,8 g (0,027 Mol) Natriumhydrid in das Gemisch, läßt 10 Minuten nachrühren, erwärmt langsam auf Rückflußtemperatur und läßt 10 Stunden nachrühren. Danach läßt man das Reaktionsgemisch auf Raumtemperatur abkühlen, gießt auf Wasser, extrahiert mit Dichlormethan und engt die organische Phase unter vermindertem Druck ein. Der Rückstand wird an Kieselgel mit einem Gemisch aus Essigester/Cyclohexan = 3 : 1 als Laufmittel chromatographiert.

Man erhält 4,0 g (69,4 % der Theorie) an 3,3-Dimethyl-2-phenyl-2-(imidazol-1-yl-methyl)-oxiran als Öl [$^1$H-NMR* : δ(1,01 (s)3H; 1,65 (s)3H)].

In analoger Weise zu Beispiel 6 und unter Berücksichtigung der Angaben zu dem entsprechenden

Verfahren werden die in der folgenden Tabelle 3 aufgeführten Verbindungen der Formel (II) hergestellt:

**Tabelle 3:**

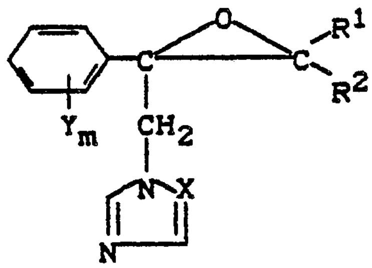

(II)

| Bsp. Nr. | $R^1$ | $R^2$ | X | 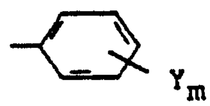 | physikal. Konstante |
|---|---|---|---|---|---|
| 7 | $CH_3$ | $CH_3$ | N | 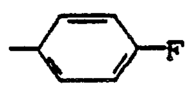 | Fp=74° C |
| 8 | $CH_3$ | $CH_3$ | N | 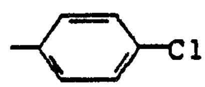 | $^1H$-NMR*):<br>δ=1,60(s);3H<br>0,93(s);3H |
| 9 | $CH_3$ | $CH_3$ | N | 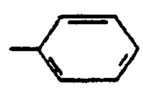 | $^1H$-NMR*):<br>δ=1,71(s);3H<br>1,05(s);3H |
| 10 | $CH_3$ | $CH_3$ | N | 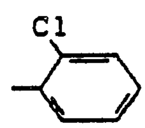 | $^1H$-NMR*):<br>δ=1,59(s);3H<br>1,01(s);3H |
| 11 | $CH_3$ | $CH_3$ | N | 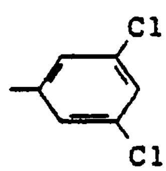 | $^1H$-NMR*):<br>δ=1,69(s);6H<br>1,08(s);1H |
| 12 | $CH_3$ | $CH_3$ | N | 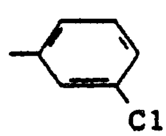 | $^1H$-NMR*):<br>δ=1,70(s);3H<br>1,06(s);3H |

*)Die $^{1}H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Verwendungsbeispiel

In dem folgenden Verwendungsbeispiel werden die nachstehend angegebenen Verbindungen als Vergleichssubstanzen eingesetzt:

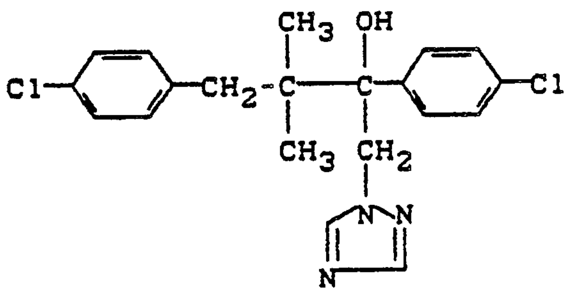
(A)

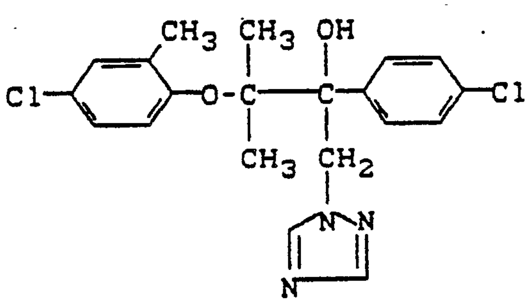
(B)

[Die Vergleichssubstanzen (A) und (B) sind bekannt aus DE-OS 32 45 504].

Beispiel A

Drechslera graminea-Test (Gerste) / Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen

im Kühlschrank 10 Tage lang einer Temperatur von 4 °C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

In diesem Test zeigt die in dem Beispiel (2) aufgeführte erfindungsgemäße Verbindung eine wesentlich bessere Wirksamkeit als die Vergleichssubstanzen (A) und (B).

## Ansprüche

1. Substituierte Vinylazole der Formel

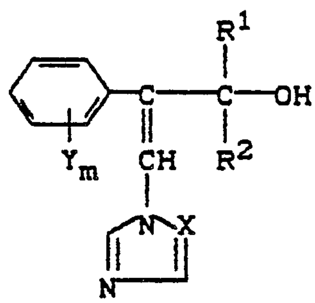

(I)

in welcher

$R^1$ für Alkyl steht,

$R^2$ für Alkyl steht,

X für ein Stickstoffatom oder die CH-Gruppe steht,

Y für Halogen, Alkyl, Cycloalkyl, Alkoxy, Alkylthio, Halogenalkyl, Halogenalkoxy, Halogenalkylthio, gegebenenfalls substituiertes Phenyl, gegebenenfalls substituiertes Phenoxy, gegebenenfalls substituiertes Phenylalkyl und/oder gegebenenfalls substituiertes Phenylalkoxy steht und

m für die Zahlen 0, 1, 2 oder 3 steht,

sowie deren Säureadditions-Salze und Metallsalz-Komplexe.

2. Substituierte Vinylazole der Formel (I) gemäß Anspruch 1, in denen

$R^1$ für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht,

X für ein Stickstoffatom oder die CH-Gruppe steht,

Y für Halogen, Alkyl mit 1 bis 4 Kohlenstoffatomen, Cycloalkyl mit 3 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, insbesondere Fluor- und/oder Chloratomen, Halogenalkoxy mit 1 oder 2 Kohlenstoffatomen und 1 bis 5 gleichen oder verschiedenen Halogenatomen, insbesondere Fluor- und/oder Chloratomen, Halogenalkylthio mit 1 oder 2 Kohlenstoffatomen und 1 bis 5 gleichen oder verschiedenen Halogenatomen, insbesondere Fluor-und/oder Chloratomen, sowie für gegebenenfalls durch Halogen und/ oder Alkyl mit 1 oder 2 Kohlenstoffatomen substituiertes Phenyl, gegebenenfalls durch Halogen und/ oder Alkyl mit 1 oder 2 Kohlenstoffatomen substituiertes Phenoxy, gegebenenfalls durch Halogen und/oder Alkyl mit 1 oder 2 Kohlenstoffatomen substituiertes Phenylalkyl mit 1 oder 2 Kohlenstoffatomen im Alkylteil und/oder für gegebenenfalls durch Halogen und/oder Alkyl mit 1 oder 2 Kohlenstoffatomen substituiertes Phenylalkoxy mit 1 oder 2 Kohlenstoffatomen im Alkoxyteil steht, und

m für die Zahlen 0, 1, 2 oder 3 steht.

3. Verfahren zur Herstellung von substituierten Vinylazolen der Formel

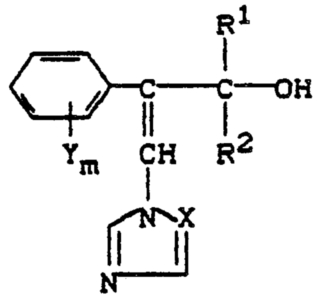

(I)

in welcher
$R^1$ für Alkyl steht,
$R^2$ für Alkyl steht,
X für ein Stickstoffatom oder die CH-Gruppe steht,
Y für Halogen, Alkyl, Cycloalkyl, Alkoxy, Alkylthio, Halogenalkyl, Halogenalkoxy, Halogenalkylthio, gegebenenfalls substituiertes Phenyl, gegebenenfalls substituiertes Phenoxy, gegebenenfalls substituiertes Phenylalkyl und/oder gegebenenfalls substituiertes Phenylalkoxy steht und
m für die Zahlen 0, 1, 2 oder 3 steht,
sowie von deren Säureadditions-Salzen und Metallsalz-Komplexen, dadurch gekennzeichnet, daß man Oxirane der Formel

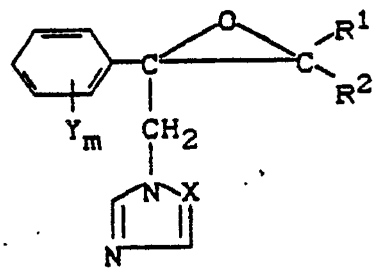

(II)

in welcher
$R^1$, $R^2$, X, Y und m die oben angegebene Bedeutung haben,
mit Wasser gegebenenfalls in Gegenwart eines Verdünnungsmittels und in Gegenwart starker Säuren umsetzt und gegebenenfalls anschließend an die so erhaltenen Verbindungen der Formel (I) eine Säure oder ein Metallsalz addiert.

4. Fungizide Mittel, gekennzeichnet durch einen Gehalt an mindestens einem substituierten Vinylazol der Formel (I) gemäß Anspruch 1 bzw. an einem Säureadditions-Salz oder Metallsalz-Komplex eines substituierten Vinylazols der Formel (I).

5. Verwendung von substituierten Vinylazolen der Formel (I) gemäß Anspruch 1 bzw. von deren Säureadditions-Salzen und Metallsalz-Komplexen zur Bekämpfung von Pilzen.

6. Verfahren zur Bekämpfung von Pilzen, dadurch gekennzeichnet, daß man substituierte Vinylazole der Formel (I) gemäß Anspruch 1 bzw. deren Säureadditions-Salze oder Metallsalz-Komplexe auf die Pflanzen und/oder deren Lebensraum ausbringt.

7. Verfahren zur Herstellung von fungiziden Mitteln, dadurch gekennzeichnet, daß man substituierte Vinylazole der Formel (I) gemäß Anspruch 1 bzw. deren Säureadditions-Salze oder Metallsalz-Komplexe mit Streckmitteln und/oder oberflächenaktiven Stoffen vermischt.